**Europäisches Patentamt**

**European Patent Office**

**Office européen des brevets**

(19)

(11) Veröffentlichungsnummer: **0 141 939**
A2

(12) # EUROPÄISCHE PATENTANMELDUNG

(21) Anmeldenummer: **84110045.6**

(51) Int. Cl.⁴: **G 01 N 33/531**, G 01 N 33/555

(22) Anmeldetag: **23.08.84**

(30) Priorität: **30.08.83 CH 4753/83**

(71) Anmelder: **Giannitsis, Dimitrios, Am Steinhuebel 11B, D-6650 Homburg (DE)**

(43) Veröffentlichungstag der Anmeldung: **22.05.85 Patentblatt 85/21**

(72) Erfinder: **Der Erfinder hat auf seine Nennung verzichtet**

(74) Vertreter: **Schulze, Ilse, Dipl.-Chem. et al, Patenanwälte Dipl.-Chem. I. Schulze Dipl.-Ing. E. Gutscher Gaisbergstrasse 3, D-6900 Heidelberg (DE)**

(84) Benannte Vertragsstaaten: **AT DE FR IT**

(54) **Verfahren zur Herstellung von natürlichem Immunoadsorbens.**

(57) Es wird ein natürliches Immunoadsorbens hergestellt, indem die Membranen von humanen oder tierischen Erythrozyten, Leukozyten und/oder Thrombozyten isoliert und anschliessend getrocknet werden. Das pulverförmige, gegebenenfalls standardisiere Immunoadsorbens kann zur Antikörperdifferenzierung, Herstellung von monospezifischen Antiseren, Entfernung von Antikörpern aus Patientenseren, Plasmafiltration und zur Herstellung von isoagglutininfreien Plasmapräparaten verwendet werden.

EP 0 141 939 A2

0141939

Verfahren zur Herstellung von
natürlichem Immunoadsorbens

*l*

Die Erfindung betrifft die Herstellung eines natürlichen Immunoadsorbens, das aus den Membranen von menschlichen oder tierischen Erythrozyten, Leukozyten und/oder Thrombozyten gewonnen wird. Das Erythrozytenstroma bzw. die Leukozyten- und Thrombozyten-Membranen in getrocknetem Zustand, nämlich in Pulverform, können zur Differenzialdiagnose unbekannter Antikörper im Blut verwendet werden. Mit diesem Adsorbens können auch monospezifische Antiseren durch Adsorptionsverfahren hergestellt werden. Die Erfindung betrifft ebenfalls Adsorptionssäulen, die das genannte Immunoadsorbens enthalten, und zur Entfernung von Antikörpern, die gegen spezifische Antigene von Erythrozyten, Leukozyten oder Thrombozyten gerichtet sind, verwendet werden können.

Verfahren zur Isolierung von Antikörpern mit Hilfe von Immunoadsorbensien, die auf dem Prinzip basieren, dass die Antikörper auf einer festen Antigenoberfläche adsorbiert werden und nach der Adsorption eluiert werden können, sind schon seit längerer Zeit bekannt. Die entsprechende klassische Arbeit von Campbell et al erschien bereits im Jahre 1951 in "Proc. Natl. Acad., U.S. 37,575". Nach chemischen Bindungsverfahren in verschiedenen Substanzen, wie beispielsweise Cellulose, können die Antigene identifiziert werden. Es sind bis heute mehrere Typen von künstlichen Immunoadsorbentien bekannt.

Die Membranen der Zellbestandteile des Blutes d.h. der Erythrozyten, Leukozyten und Thrombozyten, sowie wahrscheinlich auch andere Gewebe (Spermatozoen, Enthothelzellen usw.) sind Träger von spezifischen Antigenen, den sogenannten Blutgruppen- und Gewebeantigenen. Somit stellen die Membranen dieser Zellen natürliche Immunoadsorbensien für Antikörper dar. Diese Anwendungsmöglichkeit der Erythrozytenmembranen in Form des sogenannten Stroma ist in der Immunhämotologie schon seit langer Zeit bekannt. Landsteiner und Miller (J.exp.Med. 42, 853 - 862, 1925) haben erstmals Erythrozyten als Immunoadsorbensoberfläche angewendet, um Rh-Antikörper durch ein Adsorptions-Elutions-Verfahren zu differenzieren. Seit dieser Zeit sind zahlreiche Verfahren über die Differenzierung von Blutgruppen-Antikörpern mit Hilfe von Adsorption mit Erythrozytenstroma bekannt geworden.

Es ist ebenfalls bekannt, Blutgruppen-Antikörper durch Adsorptionsverfahren mit synthetisierten Antigenen herzustellen. (1,2) Über die Entfernung von Rh-Antikörpern mit Hilfe von Erythrozytenstroma durch chromatographische Verfahren ist wenig bekannt.

Aufgabe der vorliegenden Erfindung ist es, ein Immunoadsorbens bereitzustellen, welches frei von den Nachteilen der obengenannten Verfahren ist und welches für eine vielfältige und breite Anwendungsmöglichkeit geeignet ist. Die bisher eingesetzten Erythrozytenstroma sind nicht standartisiert, d.h. ihre Adsorptionskapazität ist nicht bekannt. Ein standartisiertes Erythrozytenstroma kann jedoch wie folgt verwendet werden:

1) Zur Differenzialdiagnose und zur quantitativen Bestimmung von erythrozyteren Antikörpern,

2) zur Behandlung von Patienten mit erythrozyteren Antikörpern, indem man dem Patientenserum die genannten Antikörper durch ein Affinitätsverfahren mit vorbereiteten Säulen entfernt (klinische Anwendung), und

3) zur Herstellung von monospezifischen Antiseren.

Diese Anwendungsmöglichkeiten gelten ebenfalls für Leukozyten und Thrombozyten.

Gegenstand der vorliegenden Erfindung ist somit das im Anspruch 1 beschriebene Verfahren zur Herstellung von Immunoadsorbens, eine Adsorptionssäule, enthaltend das Immunoadsorbens gemäss Anspruch 1, wie in Anspruch 8 beschrieben und ein Verfahren zur Herstellung von Antikörpern, die gegen spezifische Antigene von Erythrozyten, Leukozyten oder Thrombozyten gerichtet sind, wie in Anspruch 9 beschrieben.

Das erfindungsgemässe Immunoadsorbens hat den Vorteil, dass es nicht nur einmal verwendbar ist. Es kann durch Elution, Spülen und gegebenenfalls Sterilisieren zur weiteren Verwendung regeneriert werden. Es ist selbstverständlich, dass die Adsorbensien und die Adsorptionssäulen für die klinische Anwendung in sterilem und pyrogenfreiem Zustand sein müssen.

Beispiel
a) Verfahren zur Herstellung eines standardisierten Adsorptionsmaterials

500 ml konserviertes ACD- oder CPDA-Blut werden 30 min bei 2500 Umdrehungen/min zentrifugiert. Der Überstand wird abgepresst und mit isotoner NaCl-Lösung gewaschen und anschliessend nochmals 30 min bei 2500 Umdrehungen/min zentrifugiert, wobei dieser Waschvorgang dreimal wiederholt wird. In das Sediment der letzten Zentrifugation wird 200 ml destilliertes Wasser (eventuell 200 ml destilliertes Wasser + 1 ml 25% HCl) gegeben und aufgeschlämmt. Die Aufschlämmung wird bei -18°C eingefroren und anschliessend aufgetaut und 30 min bei 2500 Umdrehungen/min zentrifugiert. Das Sediment wird

- 4 -

mit 300 ml NaCl-Lösung (0,9 Gew.%) durch Zentrifugation mehrmals gewaschen, bis das Hämoglobin entfernt ist. Das Stroma wird weiter mit Filterpapier filtriert. Das Filtrat wird zur Spontansedimentation mehrere Stunden stehengelassen. Bei guter Sedimentation wird der Überstand mit der Wasserstrahlpumpe abgesaugt. Bei nicht-sichtbarer Sedimentation wird das vollständige Filtrat weiterverarbeitet, indem es in Plastikröhrchen abgefüllt und 10 min in einer kleinen Zentrifuge bei 2000 Umdrehungen/min zentr-ifugiert wird. Der Überstand wird abgesaugt und dreimal mit NaCl gewaschen, wobei 10 min bei 2000 Umdrehungen/min zentrifugiert wird, und der Überstand mit einer Pipette abgesaugt wird.

b) <u>Herstellung von Pulver</u>

I Acetonbehandlung:
Das Stroma wird in ein Glasröhrchen gegeben (bessere Durchmischbarkeit, in Plastikröhrchen nicht möglich).

Das Stroma wird mit Aceton gewaschen und mit einem Spatel gut durchgemischt, 5 min bei 2000 Umdrehungen/min zentrifugiert und der Überstand wird abgesaugt. Das Sediment wird mehrere Tage im Exikkator zum Trocknen stehengelassen (lyophilisieren ist wegen dem Aceton nicht möglich).

II Aceton-Ether-Behandlung:
Das Stroma wird mit gleichen Teilen Aceton und Ether in einem Glasröhrchen gewaschen, gut durchmischt und anschliessend 5 min bei 2000 Umdrehungen/min zentrifugiert. Der Überstand wird abgesaugt und das Sediment wird mehrere Tage in einem Exikkator zum Trocknen stehengelassen.

III Rotationsverdampfer-Technik:

Das Erythrozytenstroma wird in einen Kolben gegeben, der an einen Rotationsverdampfer angeschlossen wird und in einem Wasserbad auf 37°C erhitzt wird, wobei die Flüssigkeit unter Rotation des Kolbens und unter reduziertem Druck während mehreren Stunden abgedampft wird, wobei ein pulverförmiger Rückstand zurückbleibt.

IV Lyophilisieren:

Hier ist keine Vorbehandlung mit Aceton oder Ether notwendig. Das Erythrozytenstroma wird in einen Kolben gegeben, in einer Kältemischung schnell eingefroren und zur Gefriertrocknung an eine Hochvakuumpumpe angeschlossen. Das gefriergetrocknete pulverförmige Material ist am besten zur Weiterverarbeitung geeignet.

c) Standardisieren des Pulvers

I Das Pulver wird genau abgewogen, wobei das Gewicht in mg ermittelt wird.

II Es wird eine Erythrozytenstromasuspension hergestellt, indem 10 mg Pulver mit 0,5 ml 0,9%ige NaCl-Lösung vermischt wird.

Anmerkung: Das lyophilisierte Pulver ist mit NaCl-Lösung am besten mischbar. Andere Mischungen müssen homogenisiert werden (eine min in Eis/Wasserbad).

III Bestimmung des Adsorptionstiters:

1. Antiserum (z.B. $A_1$-Antiserum) wird quantitativ mit Testerythrozyten ($A_1$) angesetzt und 15 min bei Zimmertempe-

ratur inkubiert. Nach der Inkubation wird der Titer bestimmt (Höhe des Titers bei dem keine Agglutination mehr sichtbar ist).

2. Gleiche Volumen von Antiserum ($A_1$) werden zu 1 ml Pulverlösung gegeben, 30 min bei 37°C im Wasserbad inkubiert und zentrifugiert (4 min bei 200 Umdrehungen/min).

3. Kleine Mengen des Überstandes (0,1 ml) werden mit Testerythrozyten ($A_1$, 0,05 ml) vermischt und 15 min bei Zimmertemperatur inkubiert. Anschliessend wird über einer indirekten Lichtquelle abgelesen, bei welcher Pulverkonzentration das Antiserum vollständig adsorbiert ist.

4. Die Adsorptionskapazität des Pulvers wird nach der folgenden Formel berechnet:

$$\text{Adsorptionskapazität} = \frac{\text{mg Pulver}}{\text{ml Antiserum (bzw. Plasma)}}$$

Anwendungsmöglichkeiten von Erythrozytenstroma-Pulver

I. In der serologischen Diagnostik

1. Antikörperdifferenzierung nach Adsorption an die entsprechenden Erythrozytenantigene.
a) ABO-, Rh-, MNS-, P- etc. -System.
b) HLA-System bei Leukozyten- bzw. Thrombozyten-Antigenen.
2. Herstellung von monospezifischen Antiseren (Antikörper nach Adsorptions-Elutionsverfahren.

II. In der Immunotherapie

Entfernung von Antikörpern bei Patienten durch:

a) Plasmaphorese - Adsorption der Antikörper, z.B. Anti-D bei Schwangerschaft - und Rücktransfusion des antikörperfreien eigenen Plasma an den Patienten.

b) Plasmafiltration: Das antikörperreiche Plasma lässt man durch eine Säule mit dem Immunoadsorbens fliessen, die Antikörper werden am Adsorbens adsorbiert (Säulenfiltration) und das antikörperfreie Plasma kann wieder an den Patienten pretransfundiert werden.

III. Herstellung von isoagglutininfreien Plasmapräparaten

a) Herstellung aus Poolplasma Isoagglutinin Frischprozessplasma nach Adsorption mit A- und B-Pulver-Erythrozyten.

b) Herstellung aus Poolplasma Isoagglutinin Faktor-VIII- und Faktor-IX-Präparate (gleich wie AB-Plasma).

Literatur

1). William I. Beusinger et al: Immunoadsorption for removal of A and B Blood-Group Antibodies. The new England Journal of Medicine 304, 160 - 162, 1981.

2). J.W. Eveleigh: Techniques and Instrumentations for preparative immunoadsorbent Separations. J. Chromat. 159, 129 - 145, 1978.

- 8 -

## Patentansprüche

1. Verfahren zur Herstellung von Immunoadsorbens,
   dadurch gekennzeichnet, dass humane oder tierische Erythrozyten-, Leukozyten- und/oder Thrombozyten-Membranen isoliert
   und diese zu einem Pulver getrocknet werden.

2. Verfahren nach Anspruch 1, dadurch gekennzeichnet, dass
   die Erythrozyten-, Leukozyten- und/oder Thrombozyten-Membranen durch folgende Schritte isoliert werden:
   a) Abtrennen der Zellbestandteile aus einer ACD- oder CPDA-
      Blutkonserve durch Zentrifugation,
   b) Zugabe von gegebenenfalls leicht mit HC1 angesäuertem
      Wasser zum erhaltenen gewaschenen Sediment und Aufschlämmung desselben,
   c) Einfrierung und anschliessende Auftauung des erhaltenen
      Sedimentes, und
   d) Abtrennen des Hämoglobins und der übrigen Inhaltstoffe
      der Zellbestandteile durch Zentrifugation und Waschen der
      isolierten Zellmembranen.

3. Verfahren nach Anspruch 1 oder 2, dadurch gekennzeichnet,
   dass die isolierten Zellmembranen zu einem Pulver getrocknet
   werden, indem sie vorerst mit Aceton behandelt werden und
   anschliessend während mehrerer Tage in einem Exikkator getrocknet werden.

4. Verfahren nach Anspruch 1 oder 2, dadurch gekennzeichnet, dass
   die isolierten Zellmembranen zu einem Pulver Exik-getrocknet
   werden, indem sie vorerst mit einem Gemisch, das Aceton und Ether zu gleichen Volumenteilen enthält, behandelt werden und anschliessend während mehreren Tagen in einem Exikator getrocknet werden.

5. Verfahren nach Anspruch 1 oder 2, dadurch gekennzeichnet, dass die isolierten Zellmembranen durch Abdestillieren der Flüssigkeit unter vermindertem Druck bei einer Temperatur von etwa 37°C in ein trockenes Pulver übergeführt werden.

6. Verfahren nach Anspruch 1 oder 2, dadurch gekennzeichnet, dass die isolierten Zellen durch Lyophilisation getrocknet und in die pulverförmige Form übergeführt werden.

7. Verfahren nach einem der Ansprüche 1 bis 6, dadurch gekennzeichnet, dass das Immunoadsorbens standartisiert wird, indem die Adsorptionskapazität des Pulvers bestimmt wird.

8. Adsorptionssäulen zur Entfernung von Antikörpern, die gegen spezifische Antigene von Erythrozyten, Leukozyten oder Thrombozyten gerichtet sind, aus Patientenseren, dadurch gekennzeichnet, dass die Säule mit einem Immmunoadsorbens, hergestellt nach Anspruch 1, gefüllt ist.

9. Verfahren zur Entfernung von Antikörpern, die gegen spezifische Antigene von Erythrozyten, Leukozyten oder Thrombozyten gerichtet sind, aus Seren, dadurch gekennzeichnet, dass das Serum zur Adsorption der Antikörper mit einem Immunoadsorbens nach Anspruch 1 vorübergehend in Kontakt gebracht wird.